(19) Europäisches Patentamt
European Patent Office
Office européen des brevets

(11) EP 0 628 304 B1

(12) # FASCICULE DE BREVET EUROPEEN

(45) Date de publication et mention de la délivrance du brevet: **11.11.1998 Bulletin 1998/46**

(51) Int. Cl.[6]: **A61K 7/48**, A61K 7/06

(21) Numéro de dépôt: **94401255.8**

(22) Date de dépôt: **07.06.1994**

(54) **Composition cosmétique contenant un pseudo-latex ayant des propriétés de rémanence**
Kosmetische Zusammensetzung mit einem Pseudo-Latex mit Remanenz Eigenschaften
Cosmetic composition containing a pseudo-latex with remanence properties

(84) Etats contractants désignés:
**AT BE CH DE DK ES FR GB GR IE IT LI NL PT SE**

(30) Priorité: **08.06.1993 FR 9306827**

(43) Date de publication de la demande:
**14.12.1994 Bulletin 1994/50**

(73) Titulaire: **L'OREAL**
**75008 Paris (FR)**

(72) Inventeurs:
- **Mougin, Nathalie**
  **F-75010 Paris (FR)**
- **Mondet, Jean**
  **F-93700 Drancy (FR)**
- **Guelton, Monique**
  **F-94100 Saint Maur (FR)**
- **Piot, Bertrand**
  **F-92250 La Garenne-Colombes (FR)**
- **Dupuis, Christine**
  **F-75018 Paris (FR)**
- **Cauwet, Danielle**
  **F-75011 Paris (FR)**

(74) Mandataire:
**Stalla-Bourdillon, Bernard**
**NONY & ASSOCIES,**
**29, rue Cambacérès**
**75008 Paris (FR)**

(56) Documents cités:
**EP-A- 0 138 395**
**EP-A- 0 214 626**
**EP-A- 0 370 764**
**EP-A- 0 418 676**
**EP-A- 0 539 251**
**WO-A-89/12438**
**FR-A- 2 697 160**
**GB-A- 1 049 063**
**GB-A- 2 034 724**
**GB-A- 2 148 714**
**US-A- 3 850 178**
**US-A- 4 960 814**

- **INT. J. PHARM. vol. 77, no. 2-3 , 1991 pages 211 - 219 H. IBRAHIM ET AL. 'Concept and development of ophthalmic pseudo-latexes triggered by pH'**
- **CHEMICAL ABSTRACTS, vol. 97, no. 18, Novembre 1982, Columbus, Ohio, US; abstract no. 150584k, 'Polymer latexes in cosmetic eye liners' page 382 ; & JP-A-57 063 315 (TEIJIN LTD)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 628 304 B1

## Description

La présente invention a pour objet une composition cosmétique destinée à une application topique ou capillaire contenant un pseudo-latex.

Plus particulièrement, la présente invention a pour objet des compositions cosmétiques sous forme de shampooing, d'après-shampooing, d'une lotion ou d'un gel coiffant ou traitant, d'un produit de mise en forme des cheveux ou encore sous forme d'un produit de maquillage tel qu'un mascara ou un vernis à ongles.

Il est d'usage courant d'utiliser dans de nombreuses formulations cosmétiques, une proportion variable, selon la nature de la formulation, d'au moins une substance filmogène permettant de conférer d'une part, aux cheveux, plus de tenue et de douceur et d'autres part aux ongles un film plus brillant et dur, adhérant parfaitement sur les ongles. Il convient toutefois que la substance filmogène présente une bonne affinité vis-à-vis de la kératine des cheveux et des ongles.

En d'autres termes, la substance filmogène, une fois la composition appliquée, doit présenter des propriétés de rémanence, c'est-à-dire être difficilement éliminable de son support par un simple lavage à l'eau ou à l'aide d'un shampooing.

Après diverses études sur un très grand nombre de polymères filmogènes, on vient maintenant de constater qu'il était possible d'obtenir d'excellentes propriétés de rémanence dans diverses compositions cosmétiques en utilisant des pseudo-latex.

Comme ceci est bien connu, et telle qu'elle sera utilisée ci-après, l'expression "pseudo-latex" désigne une suspension constituée de particules généralement sphériques d'un polymère, celles-ci étant obtenues par dispersion du polymère dans une phase aqueuse appropriée.

L'expression "pseudo-latex" ne doit pas être confondue avec l'expression "latex" ou "latex synthétique" qui est également une suspension constituée de particules d'un polymère qui sont obtenues directement par polymérisation d'un ou plusieurs monomères dans une phase aqueuse appropriée.

La présente invention a pour objet à titre de produit industriel nouveau, une composition cosmétique destinée à une application topique ou capillaire, celle-ci contenant dans un support cosmétique approprié, un pseudo-latex constitué de particules d'un polymère filmogène radicalaire à fonctions acides carboxyliques neutralisées à un taux de neutralisation compris entre 10 et 80% à l'aide d'un agent neutralisant multifonctionnel constitué soit d'une diamine soit de l'association d'un sel de métal polyvalent et d'une base organique ou minérale, le diamètre moyen desdites particules étant compris entre 10 et 450nm.

Les polymères filmogènes radicalaires à fonctions acides carboxyliques du pseudo-latex sont obtenus à partir de monomères insaturés par réaction radicalaire et ont de préférence un poids moléculaire moyen compris entre 1 000 et $10^6$ mesuré par exemple en chromatographie d'exclusion stérique.

Ces polymères sont insolubles dans l'eau et sont pour l'essentiel des polymères filmogènes radicalaires couramment utilisés pour la réalisation de compositions cosmétiques.

Parmi ces polymères filmogènes radicalaires à fonctions acides carboxyliques on peut notamment citer :

- le copolymère acétate de vinyle/acide crotonique polyoxyéthyléné vendu par la Société Hoechst sous la dénomination de "Aristoflex A", d'indice d'acide 56,
- le copolymère acétate de vinyle/acide crotonique (90/10) vendu par la société BASF sous la dénomination de "Luviset CA66", d'indice d'acide 74,
- le terpolymère acétate de vinyle/acide crotonique/néodécanoate de vinyle vendu par la Société National Starch sous la dénomination de "Résine 28-29-30", d'indice d'acide 65,
- le copolymère N-octylacrylamide/méthacrylate de méthyle/méthacrylate d'hydroxypropyle/acide acrylique/ méthacrylate de tert-butylamino éthyle vendu par la Société National Starch sous la dénomination de "Amphomer", d'indice d'acide 137,
- le copolymère alterné méthylvinyléther/anhydride maléique (50/50) monoestérifié par le butanol vendu par la Société GAF sous la dénomination de "Gantrez ES 425", d'indice d'acide 260,
- le terpolymère acide acrylique/acrylate d'éthyle/N-tert-butylacrylamide vendu par la Société BASF sous la dénomination de "Ultrahold 8", d'indice d'acide 62.

On peut également utiliser selon l'invention d'autres types de polymères filmogènes à fonctions acides carboxyliques tels que ceux décrits dans le brevet français n° 78 30596 (2.439.798) ayant la formule générale suivante :

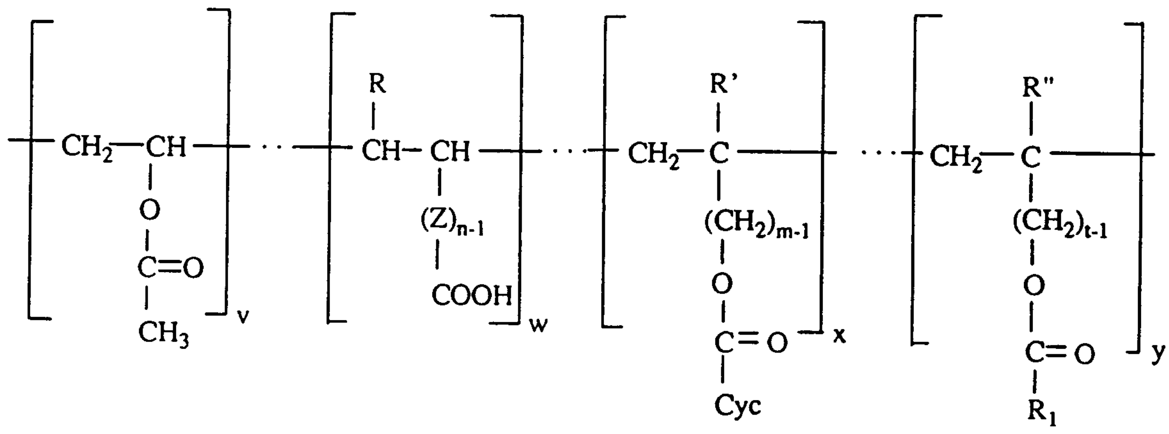

dans laquelle :

R, R' et R", identiques ou différents, représentent un atome d'hydrogène ou un radical méthyle,
m, n et t sont 1 ou 2,
$R_1$ représente un radical alkyle, linéaire ou ramifié, saturé ou insaturé ayant de 2 à 21 atomes de carbone,
Z représente un radical divalent pris dans le groupe constitué par : $-CH_2-$, $-CH_2-O-CH_2-$ et $-CH_2-O-(CH_2)_2-$,
Cyc représente un radical choisi parmi :

(i) un radical de formule :

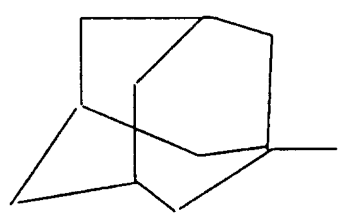

(ii) un radical de formule :

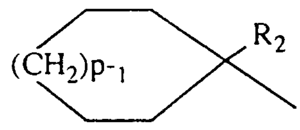

dans laquelle :

$R_2$ représente un atome d'hydrogène ou un radical méthyle,
et p est 1 ou 2,

(iii) un radical de formule :

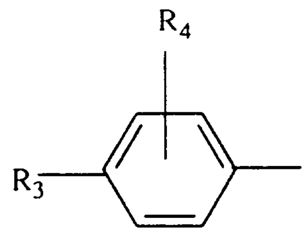

dans laquelle :

$R_3$ représente un atome d'hydrogène, un radical méthyle, éthyle, tert-butyle, éthoxy, butoxy ou dodécyloxy et $R_4$ représente un atome d'hydrogène, un radical alkyle de 1 à 4 atomes de carbone ou un radical alkoxy de 1 à 4 atomes de carbone,

et (iv) un radical de formule :

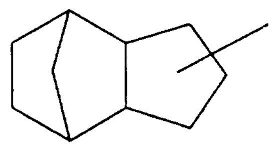

v représente de 10 à 91% et de préférence de 36 à 84% en poids,
w représente de 3 à 20% et de préférence de 6 à 12% en poids,
x représente de 4 à 60% et de préférence de 6 à 40% en poids,
et y représente de 0 à 40% et de préférence de 4 à 30% en poids,
v + w + x + y étant égal à 100%.

Le pseudo-latex des compositions cosmétiques selon l'invention est obtenu selon les méthodes connues de préparation des pseudo-latex sous réserve toutefois de certaines particularités qui seront mentionnées ci-après.

Le procédé général de préparation des pseudo-latex consiste à dissoudre un polymère insoluble dans l'eau dans un solvant organique, soluble ou partiellement soluble dans l'eau, à disperser sous agitation la solution ainsi obtenue dans de l'eau et à procéder ensuite à l'élimination du solvant organique par évaporation sous vide ce qui conduit à une suspension constituée de particules du polymère dont la taille est généralement inférieure à 1 μm.

Selon ce procédé général, l'emploi d'un tensio-actif, d'un mélange de tensio-actifs ou d'un polymère colloïde protecteur ou encore d'un mélange tensio-actif/polymère colloïde protecteur est indispensable, en vue d'obtenir une bonne stabilisation des particules.

Comme mentionné ci-dessus, les polymères filmogènes radicalaires à fonctions acides carboxyliques tels que précédemment définis doivent être neutralisés pour la préparation des pseudo-latex à un taux de neutralisation inférieur à 100% en vue d'éviter leur totale solubilisation dans l'eau.

Par une neutralisation partielle des polymères, les pseudo-latex obtenus sont particulièrement stables en l'absence de stabilisant hydrophile ou de tensio-actif ou encore de colloïde protecteur.

Le taux de neutralisation des polymères filmogènes à fonctions acides carboxyliques doit donc être parfaitement déterminé de telle sorte qu'ils restent insolubles dans l'eau tout en étant solubles dans le solvant organique.

Il va de soi que le taux limite supérieur de neutralisation qu'il conviendra de ne pas excéder pour que le polymère reste insoluble dans l'eau sera fonction de la nature de chaque polymère filmogène à fonctions acides carboxyliques et du neutralisant. De façon générale, lorsque le neutralisant est une diamine, le taux de neutralisation est généralement compris entre 30 et 80% et de préférence entre 40 et 70% si le polymère a moins de 2meq/g de fonctions acides carboxyliques et entre 10 et 50% de préférence entre 10 et 40% si le polymère a plus de 2meq/g de fonctions acides carboxyliques.

Lorsque l'agent neutralisant multifonctionnel est constitué de l'association d'un sel de métal polyvalent et d'une base minérale ou organique, le polymère est d'abord neutralisé à un taux compris entre 4 et 20%, et de préférence entre 4 et 10% par le sel de métal polyvalent, puis est co-neutralisé avec la base minérale ou organique à un taux de

neutralisation totale compris entre 30 et 80%, de préférence entre 40 et 70% si le polymère a moins de 2 meq/g de fonctions acides carboxyliques et entre 10 et 50%, de préférence entre 10 et 40 %, si le polymère a plus de 2 meq/g de fonctions acides carboxyliques.

Selon l'invention, les diamines en tant qu'agents neutralisants sont choisies parmi la lysine, l'arginine ou la cystine. Les sels de métaux polyvalents sont choisis parmi les bromures, chlorures, nitrates, acétates, carbonates et sulfates de calcium, zinc, magnésium, baryum, aluminium et zirconium.

Les bases minérales ou organiques utilisées comme co-neutralisants avec les sels de métaux polyvalents sont choisies par exemple parmi la soude, la potasse ou l'ammoniaque ou parmi un aminoalcool choisi parmi l'amino-2 méthyl-2 propanol-1 (AMP), la triéthanolamine, la triisopropanolamine (TIPA), la monoéthanolamine, la diéthanolamine, la tri[(hydroxy-2)propyl-1]amine, l'amino-2 méthyl-2 propanediol-1,3 (AMPD) et l'amino-2 hydroxyméthyl-2 propanediol-1,3.

Le co-neutralisant peut bien entendu être également une diamine telle que mentionnée ci-dessus.

Le procédé de préparation du pseudo-latex, utilisé selon l'invention est fonction de la nature de l'agent neutralisant multifonctionnel.

Lorsque l'agent neutralisant est une diamine telle que la lysine, la neutralisation des fonctions acides carboxyliques du polymère filmogène est réalisée soit in situ dans la solution du polymère dans le solvant organique par addition de la quantité déterminée de la diamine, soit lors de la préparation de l'émulsion, le neutralisant se trouvant alors dans la phase aqueuse de l'émulsion. Le solvant organique utilisé doit être un solvant volatil ou un mélange de tels solvants présentant un point d'ébullition inférieur à celui de l'eau et être miscible ou partiellement miscible à l'eau.

Le solvant organique tel que défini ci-dessus est de préférence choisi parmi l'acétone, la méthyléthylcétone, le tétrahydrofuranne, l'acétate de méthyle, l'acétate d'éthyle, l'isopropanol et l'éthanol.

Après l'obtention de la solution du polymère partiellement neutralisé dans le solvant organique, on procède alors à la préparation d'une émulsion en versant sous agitation, à la solution organique obtenue, une quantité appropriée d'eau contenant éventuellement un agent anti-mousse dont le rôle sera de faciliter l'évaporation ultérieure de la phase organique.

Selon une variante du procédé tel que défini ci-dessus la neutralisation des fonctions acides carboxyliques dit polymère en solution dans le solvant organique peut être réalisée lors de la formation de l'émulsion en y versant une solution aqueuse contenant la quantité requise de la diamine.

Lors de la formation de l'émulsion l'agitation est de préférence réalisée à l'aide d'un disperseur cisaillant du type Moritz ou Ultra-Turrax ou Raineri équipé de pales défloculantes.

L'émulsion ainsi obtenue est particulièrement stable sans qu'il soit nécessaire d'employer un agent tensio-actif dans la mesure où les groupes carboxylates du polymère se placent à l'interface avec l'eau et protègent les gouttelettes de la coalescence par répulsion électrostatique.

Après formation de l'émulsion il une température comprise entre la température ambiante et 70°C environ, on procède alors à l'évaporation sous pression réduite du solvant organique jusqu'à son élimination totale, l'évaporation étant de préférence réalisée sous léger chauffage.

On obtient ainsi un pseudo-latex, c'est-à-dire une dispersion aqueuse de particules du polymère filmogène, qui est exempt de tout tensio-actif ou autre stabilisant hydrophile tout en étant très stable.

Lorsque l'agent neutralisant multifonctionnel est constitué de l'association d'un sel de métal polyvalent et d'une base minérale ou organique, le procédé de préparation du pseudo-latex est réalisé en deux temps dans des conditions opératoires similaires. Dans un premier temps, on effectue en milieu solvant organique, la neutralisation partielle des fonctions acides carboxyliques du polymère à l'aide du sel de métal polyvalent puis dans un deuxième temps, on ajoute la base en tant que co-neutralisant en vue d'obtenir une dispersion. En effet, la neutralisation du polymère par la base puis par le sel de métal polyvalent ne permet pas d'obtenir une dispersion mais provoque lit décantation du polymère.

Selon un mode de réalisation préféré, le sel de métal polyvalent est un sel de zinc et de préférence l'acétate de zinc.

L'utilisation selon l'invention des agents neutralisants multifonctionnels permet des interactions entre les chaînes polymériques conduisant à la formation d'un réseau réticulé lors de la filmification, ce qui explique la plus grande insolubilité vis-à-vis de l'eau, des shampooings et des détergents.

La taille moyenne des particules des pseudo-latex est de préférence comprise entre 20 et 430nm.

La polydispersité en taille des particules est relativement faible selon ce procédé de préparation du pseudo-latex, celle-ci mesurée en diffusion quasi élastique de lumière est généralement comprise entre 0,1 et 0,40 et de préférence inférieure à 0,35.

Lors de la préparation, on peut introduire dans la solution du solvant organique un agent plastifiant en une proportion comprise entre 5 et 40% et de préférence entre 10 et 30% en poids par rapport au poids du polymère filmogène non neutralisé, ceci en vue d'améliorer les propriétés cosmétiques et mécaniques, ce plastifiant pouvant être hydrophile ou hydrophobe. Parmi les plastifiants hydrophiles on peut citer les éthers de glycol et en particulier :

- les Carbitols de la Société Union Carbide à savoir le Carbitol ou diéthylène glycol éthyléther, le méthyl Carbitol ou diéthylène glycol méthyléther, le butyl Carbitol ou diéthylène glycol butyléther ou encore l'hexyl Carbitol ou diéthylène glycol hexyléther,
- les Cellosolves de la Société Union Carbide à savoir le Cellosolve ou éthylène glycol éthyléther, le butyl Cellosolve ou éthylène glycol butyléther, l'hexyl Cellosolve ou éthylène glycol hexyléther,
- les Dowanols de la Société Dow Chemical et en particulier le Dowanol PM ou propylène glycol méthyléther, le Dowanol DPM ou dipropylène glycol méthyléther, le Dowanol TPM ou tripropylène glycol méthyléther ou encore le Dowanol DM ou diéthylène glycol méthyléther.

Comme autres agents plastifiants hydrophiles on peut également citer :

- l'huile de ricin oxyéthylénée à 40 moles d'oxyde d'éthylène telle que celle vendue sous la dénomination de "Mulgofen EL-719" par la Société Rhône Poulenc,
- l'alcool benzylique,
- le citrate de triéthyle vendu par la Société Pfizer sous la dénomination de "Citroflex-2",
- le 1,3-butylène glycol,
- le carbonate de propylène,
- le diéthanolamide de l'acide laurique vendu par la Société Mona Industries sous la dénomination de "Monamid 716",
- le tartrate de diéthyle,
- le phosphate de diéthyle, et
- le diacétate de glycérol (diacétine).

Parmi ces différents agents plastifiants hydrophiles on préfère utiliser dans la préparation des pseudo-latex, les Dowanols de la Société Dow Chemical.

Parmi les agents plastifiants hydrophobes on peut citer :

- les phtalates et adipates de diéthyle, de dibutyle et de diéthyl-2 hexyle,
- le tartrate de dibutyle,
- les phosphates de dibutyle et de diéthyl-2 hexyle,
- les dérivés de propylène glycol choisis parmi : le propylène glycol phényléther, le propylène glycol diacétate, le dipropylène glycol butyléther, le tripropylène glycol butyléther,
- les esters de glycérol tels que le triacétate de glycérol (triacétine).

La concentration en poids du polymère filmogène sous forme de particules dans le pseudo-latex obtenu par le procédé tel que décrit ci-dessus est généralement comprise entre 5 et 50% et de Préférence entre 10 et 25% par rapport au poids total du pseudo-latex.

La viscosité des pseudo-latex à une concentration de 25% en poids de particules de polymère filmogène doit de préférence être comprise entre 10 et 3000 x $10^{-3}$ Pa.s (10cP et 3000cP) (mesurée au Contraves à 25°C).

Les compositions cosmétiques selon l'invention ont généralement un pH compris entre environ 7 et 7,2.

Les compositions cosmétiques selon l'invention peuvent se présenter, comme mentionné ci-dessus, sous diverses formes, par exemple de shampooings, d'après-shampooings, de lotions ou de gels pour brushing ou mises en plis, de compositions de permanente ou de défrisage, de compositions de coloration ou de décoloration. Les compositions selon l'invention peuvent également se présenter sous forme de produits de maquillage des cils et des ongles tels que des mascaras ou des vernis à ongles. Les compositions selon l'invention peuvent en outre se présenter sous forme de produits pour le soin des ongles, contenant éventuellement des principes actifs et en particulier de type hydrosoluble tels que des agents hydratants et/ou durcisseurs, ainsi que sous forme de compositions de pré-revêtement pour les ongles en vue de l'application ultérieure d'un vernis à ongles, ces compositions de pré-revêtement pouvant également contenir des principes actifs hydrosolubles ou dispersés dans la composition.

La proportion en poids des particules de polymère dans ces compositions est comprise entre 0,5 et 30% par rapport au poids total de la composition, et de préférence entre 1 et 15 %.

Les compositions de l'invention peuvent contenir des filtres solaires UV-A ou UV-B ou à bande large et être utilisées comme produits antisolaires.

Elles peuvent se présenter également sous forme de déodorants ou de compositions à usage buccal telle que bains de bouche, pâtes dentifrices.

Les compositions selon l'invention peuvent contenir des adjuvants cosmétiques conventionnels choisis parmi des corps gras, des solvants organiques, des silicones, des épaississants, des adoucissants, des filtres solaires UV-A ou UV-B ou à bande large, des agents anti-mousse, des agents hydratants, des humectants, des polymères anioniques,

non-ioniques ou amphotères ou leurs mélanges, des antiperspirants, des agents alcalinisants, des colorants, des pigments et des agents propulseurs lorsque les compositions se présentent sous forme aérosol.

Plus précisément, comme corps gras, on peut utiliser une huile ou une cire ou leur mélange, des acides gras, des alcools gras, des esters d'acides gras tels que les triglycérides d'acides gras en $C_6$ à $C_{18}$, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicones et les isoparaffines.

Parmi les cires animales, fossiles, végétales, minérales ou de synthèse, on peut notamment citer la cire d'abeille, de Caroube de Candelila, l'ozokérite, les cires microcristallines ainsi que les cires et résines de silicone.

Parmi les agents épaississants, on petit citer :

- les celluloses modifiées telles que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 4400H" par la Société Amerchol,
- la gomme de caroube, la gomme de guar, la gomme d'hydroxypropylguar, la gomme de xanthane,
- les acides polyacryliques réticulés tels que les Carbopols de la Société Goodrich, les Synthalen K, L ou M de la Société Sigma,
- les polymères poly(méth)acrylates de glycéryle, vendus sous les dénominations de "Hispagel" ou de "Lubragel" par les Sociétés Hispano Quimica ou Guardian,
- la polyvinylpyrrolidone,
- l'alcool polyvinylique,
- les polymères réticulés d'acrylamide et d'acrylate d'ammonium, vendus sous les dénominations de "PAS 5161" ou "Bozepol C" par la Société Hoechst, les polymères réticulés d'acrylamide et d'acide 2-acrylamido 2-méthylpropane sulfonique partiellement ou totalement neutralisés, vendus sous la dénomination de "Sepigel 305" par la Société Seppic, les polymères réticulés d'acrylamide et de chlorure de méthacryloyloxyéthyl triméthylammonium, vendus sous la dénomination de "Salcare SC92" par la Société Allied Colloids, ou encore,
- les homopolymères ou copolymères dérivés d'acide acrylique tel que le produit vendu sous la dénomination de "Acrysol ICS-1" par la Société Seppic,
- les latex de polyuréthanne tels que le produit vendu sous la dénomination de "DSX-1514" par la Société Henkel.

Les épaississants particulièrement préférés sont ceux vendus sous la dénomination de "Synthalen K" par la Société Sigma, de "Acrysol ICS-1" par la Société Seppic et de "DSX-1514" par la Société Henkel.

On va maintenant donner à titre d'illustration plusieurs exemples de préparations des pseudo-latex ainsi que plusieurs exemples de compositions cosmétiques.

**EXEMPLES DE PREPARATION DES PSEUDO-LATEX**

**EXEMPLE 1 :** *Préparation d'un pseudo-latex du copolymère acétate de vinyle/acide crotonique/tert-butyl-4 benzoate de vinyle (65/10/25) neutralisé par la L-lysine*

La préparation de ce copolymère est décrite à l'Exemple 19 du brevet français n° 78.30596 (2.439.798) et se présente sous forme de billes ayant un diamètre de 0,5 à 1 mm.

Dans un flacon réactionnel, on introduit successivement 44,8 g de monométhyléther de tripropylène glycol et 531,2 g d'acétone.

Le mélange est agité énergiquement à l'aide d'un agitateur mécanique et on introduit lentement 224 g du copolymère défini ci-dessus. La dissolution se fait rapidement dans le flacon fermé sans chauffage.

Lorsque la phase organique est totalement solubilisée, on adapte au flacon un disperseur du type Ultra Turrax, Moritz, ou Raineri et on agite fortement (2000 tr/min environ). On introduit alors petit à petit une phase aqueuse, pour réaliser l'émulsion, celle-ci étant constituée de 532 g d'eau permutée et de 2,55 g d'anti-mousse siliconé "Burst RSD 10"et de 19,033 g de L-lysine (quantité correspondant à 50 % de neutralisation d'après l'indice d'acide du copolymère).

On observe une diminution de la viscosité lors de l'inversion de phase. L'addition de la phase aqueuse est généralement réalisée en cinq minutes et on poursuit l'agitation 10 à 15 minutes. On obtient ainsi une émulsion translucide et stable.

L'émulsion est alors introduite dans un ballon puis concentrée sous vide à l'aide d'un évaporateur rotatif en ne dépassant pas une température de 45°C. Après élimination complète de l'acétone, on obtient une dispersion stable laiteuse, peu visqueuse de concentration en polymère filmogène de 27,27 %. La viscosité mesurée au Contraves à 20°C est de $9,5 \times 10^{-3}$ Pa.s (9,5 cP). On a mesuré la taille des particules du pseudo-latex obtenu en diffusion quasi-élastique

de lumière au Coulter Model M4 et on a obtenu les résultats suivants :

- Taille moyenne des particules : 55 nm
- Facteur de polydispersité : 0,23

**EXEMPLES 2-3-4 :**

Selon le même mode opératoire que décrit ci-dessus, à l'Exemple 1, on a également préparé d'autres pseudo-latex à partir de divers copolymères filmogènes à fonctions acides carboxyliques. Les conditions d'obtention et caractéristiques des pseudo-latex obtenus sont données dans le Tableau I ci-après.

TABLEAU I

| PSEUDO-LATEX | Exemple 2 | Exemple 3 | Exemple 4 | Exemple 5 | Exemple 6 |
|---|---|---|---|---|---|
| Polymère filmogène (Indice d'acide = Ia) | Résine 28.29.30 Ia = 65 | Luviset CA 66 Ia = 74 | Amphomer LV71 Ia = 137 | Idem Ex. 1 Ia = 65 | Idem Ex. 1 Ia = 65 |
| Neutralisant | Lysine | Lysine | Lysine | Lysine | Lysine |
| Taux de neutralisation | 50 % | 50 % | 30 % | 40 % | 40 % |
| PHASE ORGANIQUE | | | | | |
| Quantité de polymère | 75 g | 75 g | 10 g | 100 g | 100 g |
| Monométhyléther de tripropylèneglycol | 15 g | 15 g | 2 g | 10 g | 20 g |
| Solvant organique volatil | Méthyléthylcétone 425 g | Méthyléthylcétone 300 | Tétrahydrog furanne 38 | Acétone g 290 g | Acétone 280 g |
| PHASE AQUEUSE | | | | | |
| Anti-mousse siliconé | 0,85 g | 0,85 g | 0,85 g | 1,14 g | 1,14 g |
| Quantité de L-lysine | 6,15 g | 7,23 g | 1,07 g | 6,78 g | 6,78 g |
| Eau permutée | 230 g | 225 g | 17.7 g | 282 g | 272 g |
| Température de formation | 60°C | Ambiante | Ambiante | Ambiante | Ambiante |
| Concentration du polymère dans le pseudo-latex | 23,50% | 20% | 22% | 25% | 25% |
| Diamètre moyen des particules | 254 nm | 240 nm | 423 nm | 71 nm | 69 nm |
| Polydispersité en taille des particules | 0,18 | 0,25 | 0,3 | 0,22 | 0,2 |

**EXEMPLE 7 :** *Préparation d'un pseudo-latex du copolymère acétate de vinyle/acide crotonique/tert-butyl-4-benzoate de vinyle (65/10/25) neutralisé par la L-arginine*

Dans un flacon réactionnel, on introduit successivement 2,8 g de monométhyléther de tripropylène glycol et 69,2 g d'acétone.

Le mélange est agité énergiquement à l'aide d'un agitateur mécanique et on introduit lentement 28 g de copolymère acétate de vinyle/acide crotonique/tert-butyl-4-benzoate de vinyle (65/10/25) utilisé à l'exemple 1. La dissolution se fait rapidement dans le flacon fermé sans chauffage.

Lorsque la phase organique est totalement solubilisée, on adapte au flacon un disperseur Moritz et on agite fortement (2.500 tr/min environ). On introduit alors petit à petit une phase aqueuse pour réaliser l'émulsion, celle-ci étant constituée de 66,05 g d'eau permutée, de 0,32 g d'anti-mousse siliconé "Burst RSD 10" et de 2,831 g de L-arginine (quantité correspondant à 50 % de neutralisation d'après l'indice d'acide du copolymère).

L'addition de la phase aqueuse est généralement réalisée en 10 minutes et on poursuit l'agitation durant environ 15 minutes à 3.000 tr/min. On obtient ainsi une émulsion laiteuse. Puis on ajoute à celle-ci sous agitation 180 g d'eau permutée.

L'émulsion est alors introduite dans un ballon puis concentrée sous vide à l'aide d'un évaporateur rotatif.

On obtient une dispersion aqueuse de concentration en polymère filmogène de 10 %. On a mesuré la taille des particules du pseudo-latex obtenu en diffusion quasi-élastique de lumière au Coulteur Model M4 et on a obtenu les résultats suivants :

- Taille moyenne des particules : 38 nm
- Facteur de polydispersité : 0,40.

On poursuit alors l'évaporation de la dispersion aqueuse à l'aide d'un évaporateur rotatif jusqu'à une concentration finale en polymère filmogène de 27 %.

**EXEMPLE 8 :** *Préparation d'un pseudo-latex du copolymère acétate de vinyle/acide crotonique/tert-butyl-4-benzoate de vinyle (65/10/25) neutralisé par l'acétate de zinc-NaOH*

(a) On dissout 20 g de copolymère acétate de vinyle/acide crotonique/tert-butyl-4 benzoate de vinyle (65/10/25) utilisé à l'exemple 1 dans 60 g d'acétone puis ajoute, sous agitation, 2,146 g d'une solution aqueuse à 10 % en poids d'acétate de zinc dihydraté. On obtient un milieu homogène et limpide. La quantité d'acétate de zinc correspond à 4,9 % de neutralisation des groupes carboxyliques du copolymère. On ajoute ensuite à la solution organique 2,9 g d'une solution aqueuse de solide 4M, quantité qui correspond à la neutralisation de 50 % des groupes carboxyliques du copolymère.

On agite la phase organique (2000 tr/min environ) a' l'aide d'un disperseur Moritz. On introduit alors petit à petit une phase aqueuse, pour réaliser l'émulsion, celle-ci étant constituée de 60 g d'eau, de 4 g de monométhyléther de tripropylèneglycol et de 0,2 g d'anti-mousse siliconé "Burst RSD 10". Après obtention de l'émulsion, on évapore l'acétone comme décrit à l'exemple 1.

On obtient une dispersion stable de concentration en polymère filmogène de 22,4 %.

- Taille moyenne des particules : 300 nm
- Facteur de polydispersité : 0,23.

(b) On opère de la même façon qu'à l'exemple 8(a) à partir des quantités suivantes de réactifs :

| | |
|---|---|
| - Copolymère utilisé à l'exemple 1 | 20 g |
| - Acétone | 60 g |
| - Solution aqueuse à 10 % d'acétate de zinc | 3,28 g (correspondant à 7,5 % de neutralisation des fonctions acides carboxyliques) |
| - Solution aqueuse de soude à 4M | 4,35 g (correspondant à 75 % de neutralisation des fonctions acides carboxyliques) |
| - Phase aqueuse identique à celle de l'exemple 8(a) | |

On obtient une dispersion stable de concentration en polymère filmogène de 21,7 %.

- Taille moyenne des particules : 75 nm
- Facteur de polydispersité : 0,4.

**EXEMPLE 9 :** *Préparation d'un pseudo-latex du copolymère acétate de vinyle/acide crotonique/tert-butyl-4 benzoate de vinyle (65/10/25) neutralisé par l'acétate de zinc-lysine*

On opère de la même façon qu'à l'exemple 8(a) à partir des quantités suivantes de réactifs :

| | |
|---|---|
| - Copolymère utilisé à l'exemple 1 | 20 g |
| - Acétone | 60 g |
| - Solution aqueuse à 10 % d'acétate de zinc | 2,146 g (correspondant à 5 % de neutralisation des fonctions acides carboxyliques) |
| - Solution aqueuse de lysine à 50 % en poids | 2,4 g (correspondant à 50 g de neutralisation totale des fonctions acides carboxyliques) |
| - Phase aqueuse identique à celle de l'exemple 8(a) | |

On obtient une dispersion stable de concentration en polymère filmogène de 22 %.

- Taille moyenne des particules : 300 nm
- Facteur de polydispersité : 0,2.

**EXEMPLES DE COMPOSITIONS**

Dans les exemples suivants, les lettres "MA" signifient "matière active" lorsque le produit utilisé, commercial ou non, se trouve sous forme d'une solution ou d'une dispersion dans un solvant.

**EXEMPLE 1 :** *Mascara crème*

| *Phase A :* | |
|---|---|
| - Stéarate de triéthanolamine | 11 g |
| - Cire d'abeille | 5 g |
| - Cire de carnauba | 3 g |
| - Paraffine | 1 g |

| *Phase B :* | |
|---|---|
| - Oxyde de fer noir | 5 g |

| Phase C : | |
|---|---|
| - Gomme arabique | 2 g |
| - Hydroxyéthylcellulose vendue sous la dénomination de "Cellosize QP" par la Société Amerchol | 1 g |

| Phase D : | |
|---|---|
| - Pseudo-latex de l'exemple 1 | 6 g (MA) |
| - Conservateurs qs | |
| - Eau | qsp 100 g |

Ce mascara est obtenu en portant les ingrédients de la *Phase A* à 85 °C, à laquelle on ajoute la *Phase B* et l'on agite à l'aide d'une turbine.

On fait ensuite bouillir l'eau de la préparation, ajoute les conservateurs, puis à 85°C, les ingrédients de la *Phase C*.

On ajoute alors la phase aqueuse obtenue (85°C) à la Phase A (80°C) sous agitation à l'aide d'une turbine (émulsification) puis on ajoute, à 30°C, le pseudo-latex de la *Phase D* et agite à l'aide d'une pale.

**EXEMPLE 2 :** *Mascara Waterproof*

On prépare un mascara résistant à l'eau ayant la composition suivante :

| | |
|---|---|
| - Cire de paraffine | 12 g |
| - Alcool de lanoline | 15 g |
| - Amidon | 2 g |
| - Oxyde de fer | 5 g |
| - Isoparaffine | 45 g |
| - Montmorillonite | 8 g |
| - Panthénol | 3 g |
| - Pseudo-latex de l'Exemple 1 | 5 g |
| - Conservateurs qsp | |

Ce mascara est obtenu par mélange des composants de la phase grasse et des éventuels additifs liposolubles. Puis on ajoute au mélange ainsi obtenu, les charges et/ou pigments éventuels, puis le (ou les) solvant(s) organique(s) volatile(s).

Enfin, on disperse dans le mélange résultant, la phase aqueuse du pseudo-latex et les éventuels additifs et/ou ingrédients actifs hydrosolubles.

**EXEMPLES 3 à 7 :** *Vernis à ongles*

| EXEMPLES | 3 | 4 | 5 | 6 | 7 |
|---|---|---|---|---|---|
| Pseudo-latex de l'exemple 1 (MA) | 28 g | 28 g | 14 g | 5,6 g | 14 g |
| Monométhyléther de tripropylène glycol | 2,8 g | 1,4 g | 0,7 g | 0,28 g | 0,7 g |
| Dispersion acrylique (1) | - | - | 14 g | 22,4 g | - |
| Dispersion de polyuréthanne (2) | - | - | - | - | 14 g |
| Epaississant du type polyuréthanne (3) | 0,3 g | 0,35 g | 0,3 g | 0,3 g | 0,4 g |
| Antimousse (4) | 0,5 g | 0,5 g | 0,5 g | 0,5 g | 0,5 g |
| Agent de surface perfluoroalkyle (5) | 0,2 g | - | 0,2 g | - | - |
| Diméthicone copolyol butyléther (6) | - | 0,15 g | - | 0,15 g | 0,15 g |
| Pigments qs | | | | | |
| Eau qsp | 100 g | 100 g | 100 g | 100 g | 100 g |

(1) vendue sous la dénomination de "Néocryl XK51" par la Société ICI,
(2) vendue sous la dénomination de "Néorez R974" par la Société ICI,
(3) vendu sous la dénomination de "Rhéolate 255" par la Société Rheox,
(4) vendu sous la dénomination de "Emulsion SF6" par la Société Wacker,
(5) vendu sous la dénomination de "Fluorad FC143" par la Société 3M,
(6) vendu sous la dénomination de "KF355A" par la Société Shin Etsu.

*Mode opératoire* :

On mélange en une seule fois le pseudo-latex de l'Exemple 1, éventuellement en présence de la dispersion acrylique ou de polyuréthanne, avec l'épaississant et les différents additifs. On agite jusqu'à obtention d'un mélange homogène, puis on y disperse les pigments qui ont été préalablement broyés en milieu aqueux.

Après application du vernis sur l'ongle, on obtient un film brillant, lisse, dur et adhérent sur l'ongle.

**EXEMPLE 8 :** *Lotion pour cheveux*

| | |
|---|---|
| - Pseudo-latex de l'Exemple 1 | 20 g (MA) |
| - Parfum, colorant, conservateur qs | |
| - Eau déminéralisée | qsp 100 g |

Par application sur les cheveux, cette lotion donne une excellente tenue à la chevelure même après trois shampooings.

**EXEMPLE 9 :** *Gel coiffant*

| | |
|---|---|
| - Pseudo-latex de l'Exemple 2 | 10 g (MA) |
| - Acide polyacrylique réticulé vendu sous la dénomination de "Carbopol 980" par la Société Goodrich | 0,3 g |

(suite)

| | |
|---|---|
| - Parfum, colorant, conservateur qs | |
| - Eau déminéralisée qsp | qsp 100 g |

Après application sur les cheveux, ce gel donne un apport de douceur même après trois shampooings.

**EXEMPLE 10 :** *Emulsion solaire huile dans l'eau*

| | |
|---|---|
| - Mélange d'alcool cétylstéarylique et d'alcool cétylstéarylique oxyéthyléné à 33 moles d'oxyde d'éthylène vendu sous la dénomination de "Synnowax AO" par la Société Henkel | 7 g |
| - Mélange de monostéarate et de distéarate de glycéryle non autoémulsionnable | 2 g |
| - Alcool cétylique | 1,5 g |
| - Huile de silicone | 1,5 g |
| - Huile de vaseline | 15 g |
| - Acide camphorsulfonique en solution aqueuse à 35 % | 3 g (MA) |
| - Triéthanolamine | 1,8 g |
| - Pseudo-latex de l'exemple 1 | 5 g (MA) |
| - Glycérine | 20 g |
| - Parfum, conservateurs qs | |
| - Eau q.s.p. | 100 g |

**EXEMPLE 11 :** *Shampooing*

| | |
|---|---|
| - Alkyl $C_9/C_{10}/C_{11}$ (20/40/40)-polyglycoside-1,4 vendu sous la dénomination de "APG 300" par la Société Henkel à 50 % | 15 g (MA) |
| - Pseudo-latex de l'exemple 1 | 3 g (MA) |
| - HCl qs pH 6 | |
| - Conservateur, parfum qs | |
| - Eau q.s.p. | 100 g |

**EXEMPLE 12 :** *Shampooing*

| | |
|---|---|
| - Acide lauryl $C_{12}/C_{14}$ (70/30) éther carboxylique oxyéthyléné à 4,5 moles d'oxyde d'éthylène neutralisé, vendu sous la dénomination de "Akypo RLM 45" par la Société Chem.Y | 8 g (MA) |
| - N-cocoamido-éthyl N-éthoxycarboxyméthyl glycinate de sodium | 4 g |
| - Pseudo-latex de l'exemple 5 | 3 g (MA) |
| - HCl qs pH 7 | |
| - Conservateur, parfum qs | |
| - Eau q.s.p. | 100 g |

**EXEMPLE 13 :** *Lotion coiffante*

| | |
|---|---|
| - Copolymère acrylamide/acide acrylamido 2-méthyl propane sulfonique sous forme de sel de sodium en émulsion inverse à 40 % dans isoparaffine/eau vendu sous la dénomination de "Sepigel 305" par la Société Seppic | 1 g (MA) |
| - Pseudo-latex de l'exemple 1 | 3 g (MA) |
| - NaOH qs pH 7,5 | |
| - Conservateur, parfum qs | |
| - Eau q.s.p. | 100 g |

**EXEMPLE 14 :** *Lotion présentée en spray flacon pompe*

| | |
|---|---|
| - Pseudo-latex de l'exemple 4 | 8 g (MA) |
| - Parfum qs | |
| - Colorant qs | |
| - Conservateur qs | |
| - Eau déminéralisée q.s.p. | 100 g |

**EXEMPLE 15 :** *Lotion de coiffage*

| | |
|---|---|
| - Pseudo-latex de l'exemple 2 | 10 g (MA) |
| - Parfum qs | |
| - Colorant qs | |
| - Conservateur qs | |
| - Eau déminéralisée q.s.p. | 100 g |

**EXEMPLE 16 :** *Bain de bouche*

| | |
|---|---|
| - Pseudo-latex de l'exemple 1 | 2,5 g (MA) |
| - Fluorure de sodium | 0,04 g |
| - Sorbitol (70 % dans l'eau) | 8 g |
| - Alcool éthylique | 6 g |
| - Huile de ricin hydrogénée polyoxyéthylénée à 40 moles d'oxyde d'éthylène vendue sous la dénomination de "Cremophor RH 40" par la Société BASF | 2 g |
| - Saccharinate de sodium | 0,015 g |
| - Arôme qs | |
| - Eau q.s.p. | 100 g |

**EXEMPLE 17 :** *Spray déodorant*

| | |
|---|---|
| - Pseudo-latex de l'exemple 1 | 5 g (MA) |
| - 2,2,4'-trichloro-2'-hydroxy diphényl éther vendu sous la dénomination de "Irgasan DP 300" par la Société Ciba Geigy | 0,3 g |
| - Alcool éthylique | 40 g |
| - Parfum qs | |
| - Eau q.s.p. | 100 g |

Cette composition est conditionnée dans un flacon pompe pour pulvérisation.

**EXEMPLE 18 :** *Gel capillaire*

| | |
|---|---|
| - Pseudo-latex de l'exemple 1 | 5 g (MA) |
| - Acrylates/steareth-20 méthacrylate copolymère vendu sous la dénomination de "Acrysol ICS" par la Société Seppic | 2 g (MA) |
| - Parfum, colorant, conservateur qs | |
| - Eau q.s.p. | 100 g |

**EXEMPLE 19 :** *Gel capillaire*

| | |
|---|---|
| - Pseudo-latex de l'exemple 5 | 8 g (MA) |
| - Acide polyacrylique réticulé vendu sous la dénomination de "Synthalen K" par la Société Sigma | 1 g (MA) |
| - Parfum, colorant, conservateur qs | |
| - Eau q.s.p. | 100 g |

**EXEMPLE 20 :** *Gel capillaire*

| | |
|---|---|
| - Pseudo-latex de l'exemple 6 | 3 g (MA) |
| - Latex de polyuréthanne vendu sous la dénomination de "DSX 1514" par la Société Henkel | 2 g (MA) |
| - Parfum, colorant, conservateur qs | |
| - Eau q.s.p. | 100 g |

**EXEMPLE 21 :** *Gel capillaire*

| | |
|---|---|
| - Pseudo-latex de l'exemple 4 | 10 g (MA) |

(suite)

| | |
|---|---|
| - Latex de polyuréthanne vendu sous la dénomination de "DSX 1514" par la Société Henkel | 2 g (MA) |
| - Parfum, colorant, conservateur qs | |
| - Eau q.s.p. | 100 g |

**EXEMPLE 22 :** *Lotion de coiffage*

| | |
|---|---|
| - Pseudo-latex de l'exemple 8(a) | 10 g (MA) |
| - Parfum, colorant, conservateur qs | |
| - Eau déminéralisée q.s.p. | 100 g |

**EXEMPLE 23 :** *Lotion de coiffage*

| | |
|---|---|
| - Pseudo-latex de l'exemple 8(b) | 8 g (MA) |
| - Parfum, colorant, conservateur qs | |
| - Eau déminéralisée q.s.p. | 100 g |

**EXEMPLE 24 :** *Mascara*

On prépare, selon le même mode opératoire qu'à l'exemple 1, un mascara ayant la composition suivante :

| *Phase A :* | |
|---|---|
| - Stéarate de triéthanolamine | 11,8 g |
| - Cire d'abeille | 5 g |
| - Cire de Carnauba | 3 g |
| - Paraffine | 1 g |

| *Phase B :* | |
|---|---|
| - Oxyde de fer noir | 5 g |

| Phase C : | |
|---|---|
| - Gomme arabique | 2 g |
| - Hydroxyéthylcellulose vendue sous la dénomination de "Cellosize QP" par la Société Amerchol | 1,2 g |

| Phase D : | |
|---|---|
| - Pseudo-latex de l'exemple 6 | 5 g (MA) |
| - Conservateurs qs | |
| - Eau q.s.p. | 100 g |

**EXEMPLE 25 :** *Mascara*

On prépare, selon le même mode opératoire qu'à l'exemple 1, un mascara ayant la composition suivante :

| Phase A : | |
|---|---|
| - Stéarate de triéthanolamine | 12 g |
| - Cire d'abeille | 8 g |
| - Cire de Carnauba | 3 g |
| - Paraffine | 2 g |

| Phase B : | |
|---|---|
| - Oxyde de fer noir | 5 g |

| Phase C : | |
|---|---|
| - Gomme arabique | 2,5 g |
| - Hydroxyéthylcellulose vendue sous la dénomination de "Cellosize QP" par la Société Amerchol | 1,5 g |
| - Hydrolysat de kératine vendu sous la dénomination de "Kerasol" par la Société Croda | 1 g |

| Phase D : | |
|---|---|
| - Pseudo-latex de l'exemple 5 | 4 g (MA) |
| - Conservateurs qs | |
| - Eau q.s.p. | 100 g |

**EXEMPLE 26 :** *Mascara*

On prépare, selon le même mode opératoire qu'à l'exemple 1, un mascara ayant la composition suivante :

| Phase A : | |
|---|---|
| - Stéarate de triéthanolamine | 11 g |
| - Cire d'abeille | 10 g |
| - Cire de Carnauba | 2 g |
| - Paraffine | 1 g |

| Phase B : | |
|---|---|
| - Oxyde de fer noir | 6 g |

| Phase C : | |
|---|---|
| - Gomme arabique | 0,8 g |
| - Hydroxyéthylcellulose vendue sous la dénomination de "Cellosize QP" par la Société Amerchol | 2 g |
| - Hydroxyéthylcellulose réticulée à l'épichlorhydrine quaternisée par la triméthylamine vendue sous la dénomination de "Celquat SC 240" par la Société National Starch | 0,1 g |
| - Polyméthacrylate de sodium vendu sous la dénomination de "Darvan 7" par la Société Vanderbilt | 1 g |

| Phase D : | |
|---|---|
| - Pseudo-latex de l'exemple 2 | 6 g (MA) |
| - Conservateurs qs | |
| - Eau q.s.p. | 100 g |

**EXEMPLE 27 :** *Mascara*

On prépare, selon le même mode opératoire qu'à l'exemple 1, un mascara ayant la composition suivante :

| *Phase A :* | |
|---|---|
| - Stéarate de glycérol | 3 g |
| - Mélange de laurate de sorbitol et de laurate de sorbitol oxyéthyléné à 20 moles d'oxyde d'éthylène vendu sous la dénomination de "Tween 20" par la Société ICI | 3,7 g |
| - Monoesters d'acide stéarique et de sorbitane vendus sous la dénomination de "Span 60" par la Société ICI | 5,6 g |
| - Cire d'abeille | 6 g |
| - Cire de Carnauba | 1,8 g |
| - Paraffine | 7,8 g |

| *Phase B :* | |
|---|---|
| - Oxyde de fer noir | 4,5 g |

| *Phase C :* | |
|---|---|
| - Hydroxyéthylcellulose vendue sous la dénomination de "Cellosize QP" par la Société Amerchol | 1,5 g |

| *Phase D :* | |
|---|---|
| - Pseudo-latex de l'exemple 3 | 2 g (MA) |
| - Conservateurs qs | |
| - Eau q.s.p. | 100 g |

**EXEMPLE 28 :** *Mascara*

On prépare, selon le même mode opératoire qu'à l'exemple 1, un mascara ayant la composition suivante :

| *Phase A :* | |
|---|---|
| - Stéarate de triéthanolamine | 11 g |

(suite)

| *Phase A :* | |
|---|---|
| - Cire d'abeille | 5 g |
| - Cire de carnauba | 3 g |
| - Paraffine | 1 g |

| *Phase B :* | |
|---|---|
| - Oxyde de fer noir | 5 g |

| *Phase C :* | |
|---|---|
| - Gomme arabique | 2 g |
| - Hydroxyéthylcellulose vendu sous la dénomination de "Cellosize QP" par la Société Amerchol | 1 g |

| *Phase D :* | |
|---|---|
| - Pseudo-latex de l'exemple 7 | 6 g (MA) |
| - Conservateurs qs | |
| - Eau | qsp 100 g |

**EXEMPLE 29 :** *Lotion de coiffage*

| | |
|---|---|
| - Pseudo-latex de l'exemple 8 | 9 g (MA) |
| - Parfum, colorant, conservateur qs | |
| - Eau déminéralisée q.s.p. | 100 g |

**Revendications**

1. Composition cosmétique destinée à une application topique ou capillaire, caractérisée par le fait qu'elle contient dans un support cosmétique approprié, un pseudo-latex constitué de particules d'un polymère filmogène radicalaire à fonctions acides carboxyliques neutralisées à un taux de neutralisation compris entre 10 et 80 % à l'aide d'un agent neutralisant multifonctionnel constitué soit d'une diamine soit de l'association d'un sel de métal polyvalent et d'une base minérale ou organique, le diamètre moyen desdites particules étant compris entre 10 et 450 nm.

2. Composition cosmétique selon la revendication 1, caractérisée par le fait que le polymère filmogène radicalaire à fonctions acides carboxyliques a un poids moléculaire moyen compris entre 1 000 et $10^6$.

**3.** Composition cosmétique selon la revendication 1 ou 2, caractérisée par le fait que le polymère filmogène radicalaire à fonctions acides carboxyliques est choisi parmi :

- les copolymères acétate de vinyle/acide crotonique polyoxyéthylénés,
- le copolymère acétate de vinyle/acide crotonique (90/10),
- les terpolymères acétate de vinyle/acide crotonique/néodécanoate de vinyle,
- les copolymères N-octylacrylamide/méthacrylate de méthyle/méthacrylate d'hydroxypropyle/acide acrylique/méthacrylate de tert-butylaminoéthyle,
- le copolymère alterné méthylvinyléther/anhydride maléique (50/50) monoestérifié par le butanol, et
- les terpolymères acide acrylique/acrylate d'éthyle/N-tert-butylacrylamide.

**4.** Composition cosmétique selon la revendication 1 ou 2, caractérisée par le fait que le polymère radicalaire à fonctions acides carboxyliques répond à la formule générale suivante :

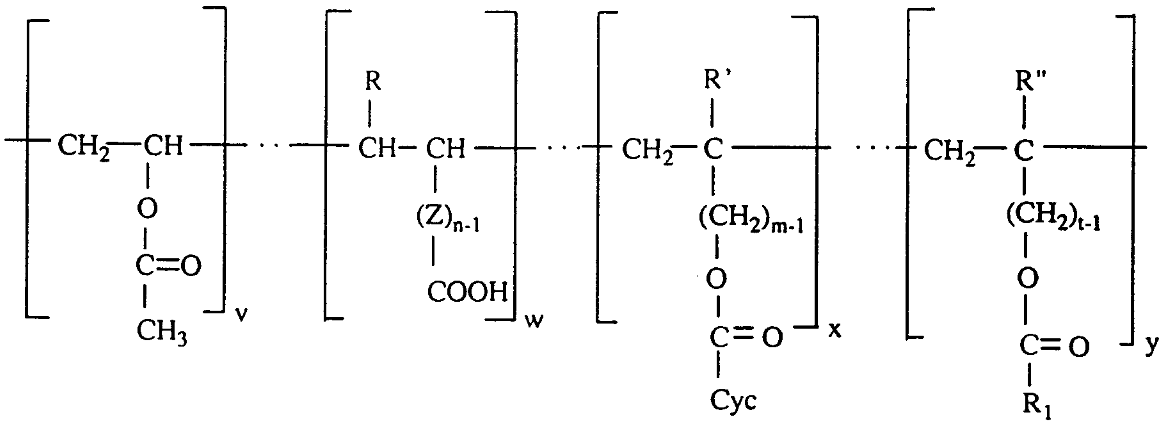

dans laquelle :

R, R' et R", identiques ou différents, représentent un atome d'hydrogène ou un radical méthyle,
m, n et t sont 1 ou 2,
$R_1$ représente un radical alkyle, linéaire ou ramifié, saturé ou insaturé ayant de 2 à 21 atomes de carbone,
Z représente un radical divalent pris dans le groupe constitué par : $-CH_2-$, $-CH_2-O-CH_2-$ et $-CH_2-O-(CH_2)_2-$,
et Cyc représente un radical choisi parmi :

(i) un radical de formule :

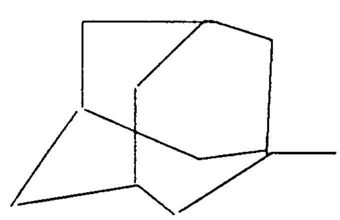

(ii) un radical de formule :

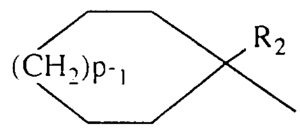

dans laquelle :

$R_2$ représente un atome d'hydrogène ou un radical méthyle, et p est 1 ou 2,

(iii) un radical de film formule :

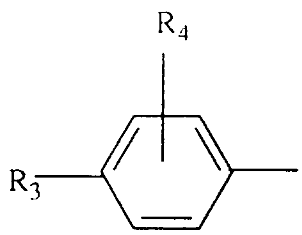

dans laquelle :

$R_3$ représente un atome d'hydrogène, un radical méthyle, éthyle, tert-butyle, éthoxy, butoxy ou dodécyloxy et $R_4$ représente un atome d'hydrogène, un radical alkyle de 1 à 4 atomes de carbone ou un radical alkoxy de 1 à 4 atomes de carbone,

et (iv) un radical de formule :

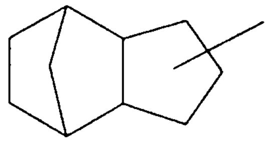

v représente de 10 à 91% et de préférence de 36 à 84% en poids,
w représente de 3 à 20% et de préférence de 6 à 12% en poids,
x représente de 4 à 60% et de préférence de 6 à 40% en poids,
et y représente de 0 à 40% et de préférence de 4 à 30% en poids,
v + w + x + y étant égal à 100%.

**5.** Composition cosmétique selon l'une quelconque des revendications précédentes caractérisée par le fait que lorsque le polymère filmogène radicalaire à fonctions acides carboxyliques est neutralisé à l'acide d'une diamine, celle-ci est choisie parmi la lysine, l'arginine et la cystine.

**6.** Composition cosmétique selon la revendication 5, caractérisée par le fait que si les fonctions acides carboxyliques du polymère filmogène ont moins de 2meq/g de fonctions acides carboxyliques le taux de neutralisation, à l'acide de la diamine, est compris entre 30 et 80% et de préférence entre 40 et 70%.

**7.** Composition cosmétique selon la revendication 5, caractérisée par le fait que si les fonctions acides carboxyliques du polymère filmogène ont plus de 2meq/g de fonctions acides carboxyliques le taux de neutralisation, à l'aide de la diamine, est compris entre 10 et 50% et de préférence entre 10 et 40%.

8. Composition cosmétique selon l'une quelconque des revendications 1 à 4, caractérisée par le fait que lorsque le polymère filmogène radicalaire à fonctions acides carboxyliques est neutralisé à l'aide de l'association d'un sel de métal polyvalent et d'une base minérale ou organique, le sel de métal polyvalent est choisi parmi les bromures, chlorures, nitrates, acétates, carbonates et sulfates de calcium, de zinc, de magnésium, de baryum, d'aluminium et de zirconium, et la base minérale ou organique est choisie parmi la soude, la potasse ou l'ammoniaque ou parmi un aminoalcool choisi parmi l'amino-2 méthyl-2 propanol-1 (AMP), la triéthanolamine, la triisopropanolamine (TIPA), la monoéthanolamine, la diéthanolamine, la tri[(hydroxy-2)propyl-1]amine, l'amino-2 méthyl-2 propanediol-1,3 (AMPD) et l'amino-2 hydroxyméthyl-2 propanediol-1,3 et les diamines.

9. Composition cosmétique selon la revendication 8, caractérisée par le fait que si les fonctions acides carboxyliques du polymère filmogène ont moins de 2 meq/g de fonctions acides carboxyliques, le polymère est neutralisé avec le sel de métal polyvalent à un taux compris entre 4 et 20 %, de préférence entre 4 et 10 %, et co-neutralisé avec la base minérale ou organique à un taux de neutralisation totale compris entre 30 et 80 % et de préférence entre 40 et 70 %.

10. Composition cosmétique selon la revendication 8, caractérisée par le fait que si les fonctions acides carboxyliques du polymère filmogène ont plus de 2 meq/g de fonctions acides carboxyliques, le polymère est neutralisé avec le sel de métal polyvalent à un taux compris entre 4 et 20 %, de préférence entre 4 et 10 %, et co-neutralisé avec la base minérale ou organique à un taux de neutralisation totale compris entre 10 et 50 % et de préférence entre 10 et 40 %.

11. Composition cosmétique selon l'une quelconque des revendications précédentes, caractérisée par le fait qu'elle contient de 0,5 à 30% en poids de particules du polymère filmogène par rapport au poids total de la composition.

12. Composition cosmétique selon l'une quelconque des revendications précédentes, caractérisée par le fait que le pseudo-latex contient un agent plastifiant en une proportion comprise entre 5 et 40% en poids par rapport au polymère filmogène.

13. Composition selon l'une quelconque des revendications précédentes, caractérisée par le fait qu'elle contient en outre au moins un additif cosmétique choisi parmi : un corps gras, un solvant organique, une silicone, un épaississant, un adoucissant, un filtre solaire UV-A ou UV-B ou à bande large, un agent anti-mousse, un agent hydratant, un humectant, un polymère anionique, non-ionique ou amphotère ou leurs mélanges, un antiperspirant, un agent alcalinisant, un colorant, un pigment ou un agent propulseur.

14. Composition selon l'une quelconque des revendications précédentes, caractérisée par le fait qu'elle contient un épaississant choisi parmi les celluloses modifiées, la gomme de caroube, la gomme de guar, la gomme d'hydroxypropylguar, la gomme de xanthane, les acides polyacryliques réticulés, les polymères poly(méth)acrylates de glycéryle, la polyvinylpyrrolidone, l'alcool polyvinylique, les polymères réticulés d'acrylamide et d'acrylate d'ammonium, les polymères réticulés d'acrylamide et d'acide 2-acrylamido 2-méthylpropanesulfonique, les polymères réticulés d'acrylamide et de chlorure de méthacryloyloxyéthyl triméthylammonium, les homopolymères ou copolymères dérivés d'acide acrylique et les latex de polyuréthanne.

**Claims**

1. Cosmetic composition intended for topical or hair application, characterized in that it contains, in a suitable cosmetic vehicle, a pseudo-latex consisting of particles of a film-forming radical polymer containing carboxylic acid functions neutralized to a degree of neutralization between 10 and 80 % using a polyfunctional neutralizing agent consisting either of a diamine or of the combination of a polyvalent metal salt and an organic or inorganic base, the average diameter of the said particles being between 10 and 450 nm.

2. Cosmetic composition according to Claim 1, characterized in that the film-forming radical polymer containing carboxylic acid functions has an average molecular weight between 1,000 and $10^6$.

3. Cosmetic composition according to Claim 1 or 2, characterized in that the film-forming radical polymer containing carboxylic acid functions is chosen from:

- polyoxyethylenated vinyl acetate/crotonic acid copolymers,
- the vinyl acetate/crotonic acid (90/10) copolymer,

- vinyl acetate/crotonic acid/vinyl neodecanoate terpolymers,
- N-octylacrylamide/methyl methacrylate/hydroxypropyl methacrylate/acrylic acid/tert-butylaminoethyl methacrylate copolymers,
- the alternating methyl vinyl ether/maleic anhydride (50/50) copolymer monoesterified with butanol, and
- acrylic acid/ethyl acrylate/N-tert-butylacrylamide terpolymers.

**4.** Cosmetic composition according to Claim 1 or 2, characterized in that the radical polymer containing carboxylic acid functions corresponds to the following general formula:

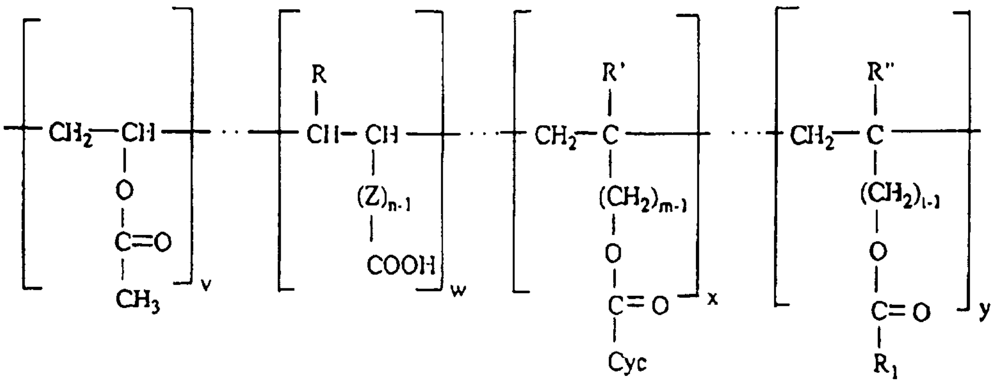

in which:

R, R' and R", which may be identical or different, represent a hydrogen atom or a methyl radical,
m, n and t are 1 or 2,
$R_1$ represents a saturated or unsaturated linear or branched alkyl radical having from 2 to 21 carbon atoms,
Z represents a divalent radical taken from the group consisting of: $-CH_2-$, $-CH_2-O-CH_2-$ and $-CH_2-O-(CH_2)_2-$,
and Cyc represents a radical chosen from:

(i) a radical of formula:

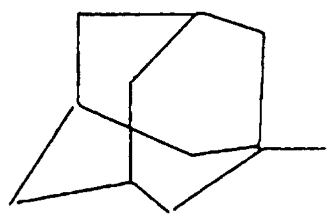

(ii) a radical of formula:

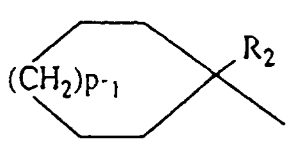

in which:

$R_2$ represents a hydrogen atom or a methyl radical,
and p is 1 or 2,

(iii) a radical of formula:

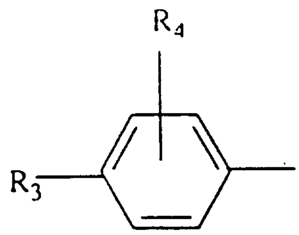

in which:

$R_3$ represents a hydrogen atom, a methyl, ethyl, tert-butyl, ethoxy, butoxy or dodecyloxy radical and $R_4$ represents a hydrogen atom, an alkyl radical of 1 to 4 carbon atoms or an alkoxy radical of 1 to 4 carbon atoms,

and (iv) a radical of formula:

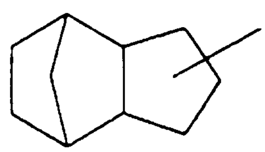

v represents from 10 to 91 % and preferably from 36 to 84 % by weight,
w represents from 3 to 20 % and preferably from 6 to 12 % by weight,
x represents from 4 to 60 % and preferably from 6 to 40 % by weight,
and y represents from 0 to 40 % and preferably from 4 to 30 % by weight,
v + w + x + y being equal to 100 %.

5. Cosmetic composition according to any one of the preceding claims, characterized in that when the film-forming radical polymer containing carboxylic acid functions is neutralized using a diamine, the latter is chosen from lysine, arginine and cystine.

6. Cosmetic composition according to Claim 5, characterized in that, if the carboxylic acid functions of the film-forming polymer have less than 2 meq/g of carboxylic acid functions, the degree of neutralization, using the diamine, is between 30 and 80 % and preferably between 40 and 70 %.

7. Cosmetic composition according to Claim 5, characterized in that, if the carboxylic acid functions of the film-forming polymer have more than 2 meq/g of carboxylic acid functions, the degree of neutralization, using the diamine, is between 10 and 50 % and preferably between 10 and 40 %.

8. Cosmetic composition according to any one of Claims 1 to 4, characterized in that, when the film-forming radical polymer containing carboxylic acid functions is neutralized using a combination of a polyvalent metal salt and an inorganic or organic base, the polyvalent metal salt is chosen from the bromides, chlorides, nitrates, acetates, carbonates and sulphates of calcium, zinc, magnesium, barium, aluminium and zirconium, and the inorganic or organic base is chosen from sodium hydroxide, potassium hydroxide or aqueous ammonia or from an amino alcohol chosen from 2-amino-2-methyl-1-propanol (AMP), triethanolamine, triisopropanolamine (TIPA), monoethanolamine, diethanolamine, tri[1-(2-hydroxy)propyl]amine, 2-amino-2-methyl-1,3-propanediol (AMPD) and 2-amino-2-hydroxymethyl-1,3-propanediol and diamines.

9. Cosmetic composition according to Claim 8, characterized in that, if the carboxylic acid functions of the film-forming polymer have less than 2 meq/g of carboxylic acid functions, the polymer is neutralized with the polyvalent metal

salt to a degree between 4 and 20 %, preferably between 4 and 10 %, and co-neutralized with the inorganic or organic base to a total degree of neutralization between 30 and 80 % and preferably between 40 and 70 %.

**10.** Cosmetic composition according to Claim 8, characterized in that, if the carboxylic functions of the film-forming polymer have more than 2 meq/g of carboxylic acid functions, the polymer is neutralized with the polyvalent metal salt to a degree between 4 and 20 %, preferably between 4 and 10 %, and co-neutralized with the inorganic or organic base to a total degree of neutralization between 10 and 50 % and preferably between 10 and 40 %.

**11.** Cosmetic composition according to any one of the preceding claims, characterized in that it contains from 0.5 to 30 % by weight of particles of the film-forming polymer relative to the total weight of the composition.

**12.** Cosmetic composition according to any one of the preceding claims, characterized in that the pseudo-latex contains a plasticizing agent in a proportion between 5 and 40 % by weight relative to the film-forming polymer.

**13.** Composition according to any one of the preceding claims, characterized in that it contains in addition at least one cosmetic additive chosen from: a fatty substance, an organic solvent, a silicone, a thickening agent, an emollient, a UV-A or UV-B or broadband sun-screen agent, an anti-foaming agent, a moisturizing agent, a humidifying agent, an anionic, non-ionic or amphoteric polymer or mixtures thereof, an anti-perspirant, a basifying agent, a dye, a pigment or a propellent.

**14.** Composition according to any one of the preceding claims, characterized in that it contains a thickening agent chosen from modified celluloses, carob gum, guar gum, hydroxypropylguar gum, xanthan gum, crosslinked polyacrylic acids, glyceryl poly(meth)acrylate polymers, polyvinylpyrrolidone, polyvinyl alcohol, crosslinked polymers of acrylamide and ammonium acrylate, crosslinked polymers of acrylamide and 2-acrylamido-2-methylpropanesulphonic acid, crosslinked polymers of acrylamide and methacryloyloxyethyltrimethylammonium chloride, homopolymers or copolymers derived from acrylic acid and polyurethane latexes.

**Patentansprüche**

**1.** Kosmetische Zubereitung, bestimmt zur topischen Anwendung oder Anwendung für das Haar, dadurch gekennzeichnet, daß sie in einem geeigneten kosmetischen Träger einen Pseudolatex enthält, der aus Teilchen eines filmbildenden radikalischen Polymers mit Carbonsäurefunktionen besteht, die mit Hilfe eines multifunktionellen Neutralisationsmittels, das entweder aus einem Diamin oder aus einer Verbindung eines Salzes eines mehrwertigen Metalls mit einer mineralischen oder organischen Base besteht, bis zu einem Neutralisationsgrad zwischen 10 und 80 % neutralisiert sind, wobei der mittlere Durchmesser der Teilchen zwischen 10 und 450 nm liegt.

**2.** Kosmetische Zubereitung nach Anspruch 1, dadurch gekennzeichnet, daß das filmbildende radikalische Polymer mit Carbonsäurefunktionen ein mittleres Molekulargewicht zwischen 1.000 und $10^6$ aufweist.

**3.** Kosmetische Zubereitung nach Anspruch 1 oder 2, dadurch gekennzeichnet, daß das filmbildende radikalische Polymer mit Carbonsäurefunktionen gewählt ist aus:

- polyethoxylierten Copolymeren aus Vinylacetat/Crotonsäure;
- Copolymeren aus Vinylacetat/Crotonsäure (90/10);
- Terpolymeren aus Vinylacetat/Crotonsäure/Vinylneodecanoat;
- Copolymeren aus N-Octylacrylamid/Methylmethacrylat/Hydroxypropylmethacrylat/ Acrylsäure/tert-Butylaminoethylmethacrylat;
- alternierenden Copolymeren aus Methylvinylether/Maleinsäureanhydrid (50/ 50), monoverestert mit Butanol; und
- Terpolymeren aus Acrylsäure/Ethylacrylat/N-tert-Butylacrylamid.

**4.** Kosmetische Zubereitung nach Anspruch 1 oder 2, dadurch gekennzeichnet, daß das radikalische Polymer mit Carbonsäurefunktionen durch die folgende allgemeine Formel wiedergegeben wird:

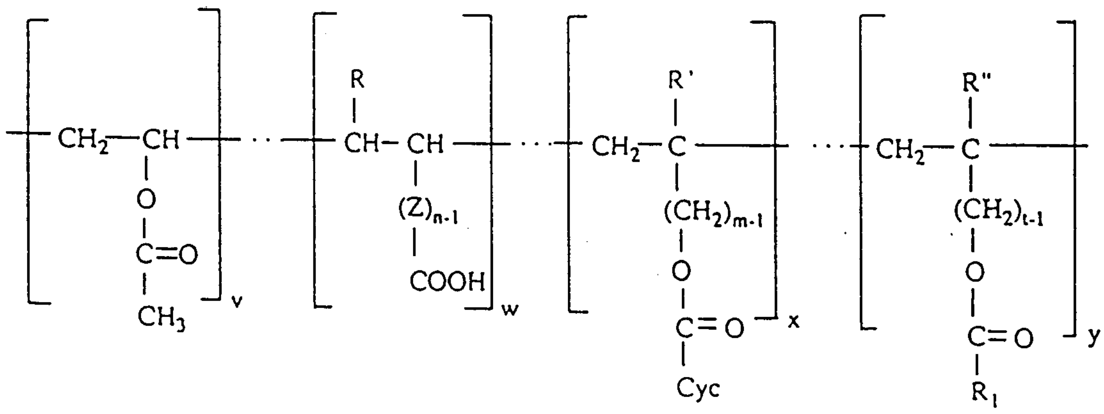

worin

R, R' und R", die gleich oder verschieden sein können, für ein Wasserstoffatom oder einen Methylrest stehen; m, n und t für 1 oder 2 stehen; $R_1$ für einen linearen oder verzweigten, gesättigten oder ungesättigten Alkylrest mit 2 bis 21 Kohlenstoffatomen steht; Z für einen zweiwertigen Rest steht, ausgewählt aus der Gruppe, die besteht aus: $-CH_2-$, $-CH_2-O-CH_2-$ und $-CH_2-O-(CH_2)_2-$; und Cyc für einen Rest steht, der gewählt ist aus:

(i) einem Rest der Formel:

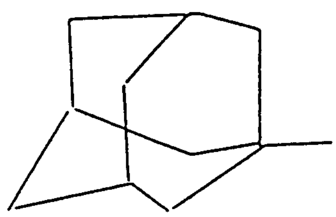

(ii) einem Rest der Formel:

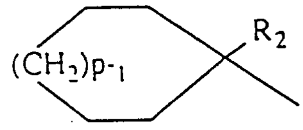

worin $R_2$ für ein Wasserstoffatom oder einen Methylrest steht und p für 1 oder 2 steht;

(iii) einem Rest der Formel:

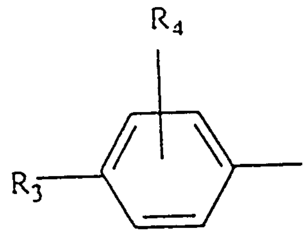

worin $R_3$ für ein Wasserstoffatom, einen Methyl-, Ethyl-, tert-Butyl-, Ethoxy-, Butoxy- oder Dodecyloxyrest steht und $R_4$ für ein Wasserstoffatom oder einen Alkylrest mit 1 bis 4 Kohlenstoffatomen oder einen Alkoxyrest mit 1 bis 4 Kohlenstoffatomen steht; und

(iv) einem Rest der Formel:

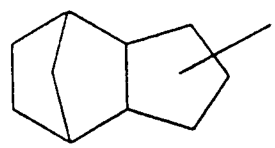

und worin
v für 10 bis 91 Gew.-%, vorzugsweise für 36 bis 84 Gew.-%, steht;
w für 3 bis 20 Gew.-%, vorzugsweise für 6 bis 12 Gew.-%, steht;
x für 4 bis 60 Gew.-%, vorzugsweise für 6 bis 40 Gew.-%, steht; und
y für 0 bis 40 Gew.-%, vorzugsweise für 4 bis 30 Gew.-%, steht; und
v + w + x + y 100 % ausmachen.

**5.** Kosmetische Zubereitung nach irgendeinem der vorangehenden Ansprüche, dadurch gekennzeichnet, daß dann, wenn das filmbildende radikalische Polymer mit Carbonsäurefunktionen mit Hilfe eines Diamins neutralisiert wird, dieses gewählt ist aus Lysin, Arginin und Cystin.

**6.** Kosmetische Zubereitung nach Anspruch 5, dadurch gekennzeichnet, daß dann, wenn die Carbonsäurefunktionen des filmbildenden Polymers weniger als 2 mÄq/g Carbonsäurefunktionen aufweisen, der Neutralisationsgrad mit Hilfe des Diamins zwischen 30 und 80 % und vorzugsweise zwischen 40 und 70 % liegt.

**7.** Kosmetische Zubereitung nach Anspruch 5, dadurch gekennzeichnet, daß dann, wenn die Carbonsäurefunktionen des filmbildenden Polymers mehr als 2 mÄq/g Carbonsäurefunktionen aufweisen, der Neutralisationsgrad mit Hilfe des Diamins zwischen 10 und 50 % und vorzugsweise zwischen zwischen 10 und 40 % liegt.

**8.** Kosmetische Zubereitung nach einem der Ansprüche 1 bis 4, dadurch gekennzeichnet, daß dann, wenn das filmbildende radikalische Polymer mit Carbonsäure-Funktionen mit Hilfe einer Verbindung eines Salzes eines mehrwertigen Metalls mit einer mineralischen oder organischen Base neutralisiert wird, das Salz des mehrwertigen Metalls gewählt ist aus Bromiden, Chloriden, Nitraten, Acetaten, Carbonaten und Sulfaten von Calcium, Zink, Magnesium, Barium, Aluminium und Zirkonium, und die mineralische oder organische Base gewählt ist aus Soda, Kali oder Ammoniak oder einem Aminoalkohol, der gewählt ist aus 2-Amino-2-methylpropanol-1 (AMP), Triethanolamin, Triisopropa-nolamin (TIPA), Monoethanolamin, Diethanolamin, Tri-[(2-hydroxy-)1-propyl-]amin, 2-Amino-2-methyl-1,3-propandiol (AMPD) und 2-Amino-2-hydroxymethyl-1,3-propandiol und Diaminen.

**9.** Kosmetische Zubereitung nach Anspruch 8, dadurch gekennzeichnet, daß dann, wenn die Carbonsäurefunktionen

des filmbildenden Polymers weniger als 2 mÄq/g Carbonsäurefunktionen aufweisen, das Polymer mit dem Salz des mehrwertigen Metalls bis zu einem Grad zwischen 4 und 20 %, vorzugsweise zwischen 4 und 10 %, neutralisiert wird und mit der mineralischen oder organischen Base bis zu einem Gesamtneutralisationsgrad zwischen 30 und 80 % und vorzugsweise zwischen 40 und 70 % coneutralisiert wird.

**10.** Kosmetische Zubereitung nach Anspruch 8, dadurch gekennzeichnet, daß dann, wenn die Carbonsäurefunktionen des filmbildenden Polymers mehr als 2 mÄq/g Carbonsäurefunktionen aufweisen, das Polymer mit dem Salz des mehrwertigen Metalls bis zu einem Grad zwischen 4 und 20 %, vorzugsweise zwischen 4 und 10 %, neutralisiert wird und mit der mineralischen oder organischen Base bis zu einem Gesamtneutralisationsgrad zwischen 10 und 50 % und vorzugsweise zwischen 10 und 40 % coneutralisiert wird.

**11.** Kosmetische Zubereitung nach einem der vorangehenden Ansprüche, dadurch gekennzeichnet, daß sie einen Gehalt von 0,5 bis 30 Gew.-% filmbildender Polymer-Teilchen aufweist, bezogen auf das Gesamtgewicht der Zubereitung.

**12.** Kosmetische Zubereitung nach einem der vorangehenden Ansprüche, dadurch gekennzeichnet, daß der Pseudolatex einen Weichmacher in einer Menge zwischen 5 und 40 Gew.-% enthält, bezogen auf das filmbildende Polymer.

**13.** Kosmetische Zubereitung nach einem der vorangehenden Ansprüche, dadurch gekennzeichnet, daß diese außerdem einen kosmetischen Zusatzstoff enthält, der gewählt ist aus einem Fettbestandteil, einem organischen Lösungsmittel, einem Silicon, einem Dickungsmittel, einem Weichmacher, einem Filter gegen UV-A- oder UV-B-Strahlung oder Strahlung in einem breiten Bereich, einem Schaumbremser, einem Feuchthaltemittel, einem Benetzungsmittel, einem anionischen, nicht-ionischen oder amphoteren Polymer oder Mischungen davon, einem Antitranspirationsmittel, einem Alkalisierungsmittel, einem Färbemittel, einem Pigment oder einem Treibmittel.

**14.** Kosmetische Zubereitung nach einem der vorangehenden Ansprüche, dadurch gekennzeichnet, daß diese ein Dickungsmittel enthält, das gewählt ist aus modifizierten Cellulosen, Carubingummi, Guargummi, Hydroxypropylguargummi` Xanthangummi, vernetzten Polyacrylsäuren, Polymeren aus Poly(meth)acrylaten und Glycerin, Polyvinylpyrrolidon, Polyvinylalkohol, vernetzten Polymeren aus Acrylamid und Ammoniumacrylat, vernetzten Polymeren aus Acrylamid und 2-Acrylamido-2-methylpropansulfonsäure, vernetzten Polymeren aus Acrylamid und Methacryloyloxyethyltrimethylammoniumchlorid, Homopolymer- oder Copolymer-Derivaten aus Acrylsäure und Polyurethanlatices.